# EUROPÄISCHE PATENTSCHRIFT

(11) **EP 0 405 575 B1**
(45) Veröffentlichungstag und Bekanntmachung des Hinweises auf die Patenterteilung: **22.09.1993**
(21) Anmeldenummer: 90112451.1
(22) Anmeldetag: 29.06.1990
(51) Int. Cl.: A61F 13/15, A41B 9/00

(54) **Verfahren zum Herstellen von Wegwerfunterhosen**
Process for manufacturing disposable panties
Procédé de fabrication de slips à jeter

(30) Priorität: 29.06.1989 JP 167224/89
(43) Veröffentlichungstag der Anmeldung: 02.01.1991
(73) Patentinhaber: UNI-CHARM CORPORATION, Kawanoe-shi Ehime-ken (JP)
(72) Erfinder: Nomura, Hironori, Iyomishima-shi, Ehime-ken (JP); Shimakawa, Taiji, Kanonji-shi, Kanagawa-ken (JP); Matsura, Yoshinori, Kanonji-shi, Kagawa-ken (JP); Yamamoto, Hiroki, Kawanoe-shi, Ehime-ken (JP); Ohnishi, Hirofumi, Iyomishima-shi, Ehime-ken (JP)
(74) Vertreter: Sperling, Rüdiger, Dipl.-Ing.

(56) Entgegenhaltungen:
- EP-A- 0 048 010
- EP-A- 0 048 011
- FR-A- 2 177 425
- US-A- 4 650 530
- US-A- 4 677 695

## Beschreibung

Die vorliegende Erfindung bezieht sich allgemein auf Wegwerfunterhosen, und insbesondere auf ein Verfahren von Windelhosen in Form von Unterhosen und Kinderübungshosen.

Verschiedene Ausführungsformen solcher Unterhosen oder kurzen Hosen sind im Stand der Technik bekannt, beispielsweise aus der EP-A-0 048 010, der EP-A-0 048 011 oder der FR-A-2 177 425. Diese Schriften offenbaren Verfahren zum Befestigen der elastischen Teile in der Taillenöffnung und den Beinöffnungen der Unterhosen, insbesondere entlang der Krümmungen der jeweiligen Beinöffnungen, um die Paßform um den Schenkel des Trägers zu verbessern.

Um sicherzustellen, daß ein ununterbrochenes elastisches Teil kontinuierlich an der Bahn an Stellen befestigt werden kann, die jeweils in der Taille und in der Beinöffnung ausgebildet sind und zwar während der Zeit, während die Grundkörper der Hosen aus dieser Bahn kontinuierlich hergestellt werden, ist es allgemein wünschenswert, daß dieses kontinuierliche elastische Teil in gedehntem Zustand in einem vorbestimmten Verhältnis gedehnt gehalten wird, wenn es auf der Bahn an den genannten Stellen unter Zuhilfenahme eines geeigneten Klebers angebracht wird. Der oben betrachtete Stand der Technik offenbart das Verfahren, mit welchem das ununterbrochene elastische Teil, das das vorher aufgetragene Klebmittel trägt, an der kontinuierlichen Bahn befestigt wird.

Allgemein ist festzustellen, daß dann, wenn das elastische Teil an der Bahn befestigt werden soll, während es gemäß der Krümmung z.B. der Beinöffnung gekrümmt wird, dieses elastische Teil danach tendiert, sich wegen seiner Zusammenziehfähigkeit in seine ursprüngliche geradlinige Ausgangslage zurückzubewegen, und es kommt daher gelegentlich vor, daß es an der Bahn an Stellen angeklebt wird, die von der gewünschten Stelle abweichen, an der das elastische Teil an der Bahn befestigt werden sollte. Diese unerwünschte Abweichung oder dieses Verschieben des elastischen Teils kann auch in dem oben erwähnten Stand der Technik geschehen, weil gemäß dieses Stands der Technik das elastische Teil, das das vorher aufgetragene Klebmittel trägt, auf ein Faservlies befestigt werden muß, dessen Oberfläche üblicherweise weniger glatt ist, als diejenige von Kunststoffilm, und der Klebeffekt ist entsprechend niedrig. Wenn das elastische Teil kontinuierlich an der Bahn befestigt wird, während es, gesteuert von in Querrichtung wirkender Mittel gekrümmt wird, um der Krümmung der Beinöffnung zu folgen, gerät eine bestimmte Menge des Klebmittels zwangsläufig auf die in Querrichtung wirkenden Mittel, wenn das elastische Teil an den in Querrichtung wirkenden Mitteln vorbeipassiert. Das hat zur Folge, daß das elastische Teil abgeschält oder verschoben wird und so die Klebwirkung zerstört wird und unerwünschterweise häufiges Säübern der in Querrichtung wirkenden Mittel insbesondere an dem Abschnitt erforderlich ist, an dem das elastische Teil vorbeigeführt wird.

Es ist daher ein erstes Ziel der Erfindung, ein Verfahren zum Herstellen von Wegwerfwindeln bereitzustellen, das es ermöglicht, das oben erwähnte Problem, das im Stand der Technik üblicherweise auftritt, zu überwinden, Gelöst wird es durch Befestigen des elastischen Teils an der Bahn entlang einer ringförmigen Klebezone an jeder Stelle, die in einer Beinöffnung auszuformen ist, wobei die ringförmige Klebezone durch einen Streifen von Klebmittel definiert ist, dessen Breite größer ist als die des elastischen Teils.

Im einzelnen wird die oben dargelegte Aufgabe erfindungsgemäß durch ein Verfahren mit folgenden Schritten gelöst:

Intermittierendes Auftragen von ringförmige Klebezonen zentral auf einer Seite einer ersten kontinuierlichen Bahn entlang ihrer Länge; Zuführen von ersten und zweiten kontinuierlichen elastischen Teilen, die eine Breite kleiner als diejenige eines Klebstreifens aufweisen, welcher die genannten ringförmigen Klebezonen in Richtung auf den Zentralbereich auf der genannten einen Seite der genannten ersten kontinuierlichen Bahn definiert, so daß das genannte erste kontinuierliche elastische Teil so aufgelegt ist, daß es entlang im wesentlichen einer Hälfte der genannten ringförmigen Klebzone anklebt, während das genannten zweite kontinuierliche elastische Teil so aufgelegt wird, daß es entlang im wesentlichen der verbleibenden Hälfte der genannten ringförmigen Klebzone anhaftet; Vorsehen von bandartigen Klebzonen, die sich entlang seitlicher Ränder auf einer Seite zumindest einer der genannten ersten und zweiten kontinuierlichen Bahn befinden; Einführen einer dritten Gruppen kontinuierlicher elastischer Teile zwischen den einander gegenüberliegenden Seiten der genannten ersten kontinuierlichen Bahn und der zweiten kontinuierlichen Bahn entlang derjeweiligen Seitenränder, so daß sie zwischen diesen Bahnen entlang der genannten bandartigen Klebezonen ankleben; verkleben der genannten ersten und zweiten kontinuierlichen Bahnen entlang der bezeichneten einen Seite, um eine kontinuierliche kombinierte Bahn zu bilden, Falten der kontinuierlichen kombinierten Bahn zu zwei Hälften entlang einer in Längsrichtung verlaufenden Zentrallinie; Wegschneiden von Abschnitten der genannten kontinuierlichen kombinierten Bahn, die in den jeweiligen ringförmigen Zonen, die diese ringförmigen Klebzonen aufweisen, eingeschlossen sind, wobei die ersten und zweiten elastischen Teile, die entlang der genannten ringförmigen Klebzone auf die Bahn geklebt sind, nach dem Formen oder Falten die genannten kombinierte Bahn doppeln, um Ausschnitte für die Beinöffnungen zu bilden; Anlegen von geradlinigen Abdichtzonen, die sich in Querrichtung der doppelt gefalteten kontinuierlichen kombinierten Bahn zentral durch die jeweiligen Ausschnitte für die Beinöffnungen zu einer Seitenkante der kombinierten Bahn erstrecken, entsprechend der Taillen-Linie, um eine kontinuierliche Serie von Unterhosen zu bilden; und Schneiden der kontinuierlichen Serie von Unterhosen entlang jeder dieser geraden Dichtzonen in Querrichtung zur kontinuierlichen Serie der Unterhosen, so daß jede der linearen Dichtzonen in Längsrichtung der genannten kontinuierlichen kombinierten Bahnen geteilt ist und auf diese Weise individuelle Unterhosen erhalten werden.

In einem bevorzugten Ausführungsbeispiel sind auf der einen Seite der zweiten kontinuierlichen Bahn zusätzlich ringförmige Klebzonen angeordnet, die jeweils identisch mit und den genannten erst erwähnten ringförmigen Klebzonen zugeordnet sind, so daß die ersten und zweiten kontinuierlichen elastischen Teile zwischen diesen gegenseitig einander gegenüberliegenden ringförmigen Klebzonen sandwichartig eingeschlossen sind.

Um Unterhosen herzustellen, die einen absorbierenden Kern aufweisen, ist ein absorbierender Kern zwischen jedem Paar der genannten ringförmigen Klebzonen eingelegt, und zwar bevor die ersten und zweiten kontinuierlichen Bahnen aufeinander geklebt werden.

Gemäß der Erfindung, wie sie oben beschrieben worden ist, sind ringförmige Klebezonen vorgesehen, die von Streifen aus Klebmitteln gebildet sind, und eine Breite aufweisen, die größer ist als diejenige der kontinuierlichen elastischen Teile und welche Streifen intermittierend entlang der Länge der Bahn aufgetragen sind, wobei die kontinuierlichen elastischen Teile für die Beinöffnungen entlang dieser ringförmigen Klebzonen befestigt sind. Der Erfolg davon ist, daß die kontinuierlichen elastischen Teile sicher und zuverlässig auf der kontinuierlichen Bahn ohne wesentliche Verschiebung oder Versetzung der kontinuierlichen elastischenTeile aus den zugeordneten ringförmigen Klebzonen erhalten wird, selbst wenn die kontinuierliche Bahn aus einem Faservlies besteht. Diese Wirkung wird verstärkt, wenn eine andere kontinuierliche Bahn mit den ringförmigen Klebezonen in Übereinstimmung mit den vorher erwähnten, jeweiligen ringförmigen Klebezonen vorgesehen wird, so daß die kontinuierlichen elastischen Teile zwischen den jeweiligen Sätzen der sich einander gegenüberliegenden ringförmigen Klebezonen sandwichartig eingeschlossen werden können.

Schließlich sind die ringförmigen Klebzonen auf der kontinuierlichen Bahn angeordnet, anstatt daß das Klebmittel unmittelbar auf den kontinuierlichen elastischen Teilen aufgetragen wird, so daß das Problem, mit dem der oben erwähnte Stand der Technik behaftet ist, bei dem das Klebmittel unmittelbar auf die kontinuierlichen elastischen Teile aufgetragen wird, gelöst wird.

Die Figurendarstellungen zeigen die folgenden Ansichten:
- Fig. 1: ist eine perspectivische Ansicht, welche Wegwerfunterhosen zeigt, die gemäß der Erfindung hergestellt und die vollständig zusammengesetzt sind;
- Fig. 2: ist eine perspektivische Explosionsdarstellung der genannten Unterhosen;
- Fig. 3: ist eine schematische diagrammartige Darstellung einer Vorrichtung zum Herstellen der genannten Unterhosen;
- Fig. 4A bis Fig. 4E und Fig. 5A bis Fig.5C: sind Teil-Draufsichten, die die jeweiligen Stufen für den Zusammenbau der genannten Unterhosen illustrieren;
- Fig. 6: ist eine Teilperspektivdarstellung, die in vergrößertem Maßstab in Querrichtung wirkende Mittel zeigen, die in dieser Vorrichtung Verwendung finden;
- Fig. 7: ist eine Teilschnittdarstellung der genannten, in Querrichtung wirkenden Mittel;
- Fig. 8: ist eine Schnittdarstellung einer in Querrichtung wirkenden Verformmechanik in diesen in Querrichtung wirkenden Mitteln; und
- Fig. 9: ist eine schematische Draufsicht, welche die elastischen Teile in deformiertem Zustand, erreicht durch die in Querrichtung wirkende Verformmechanik, zeigt.

Im folgenden wird ein Ausführungsbeispiel der Erfindung unter Bezugnahme auf die bei geschlossenen Zeichnungen im Detail erläutert.

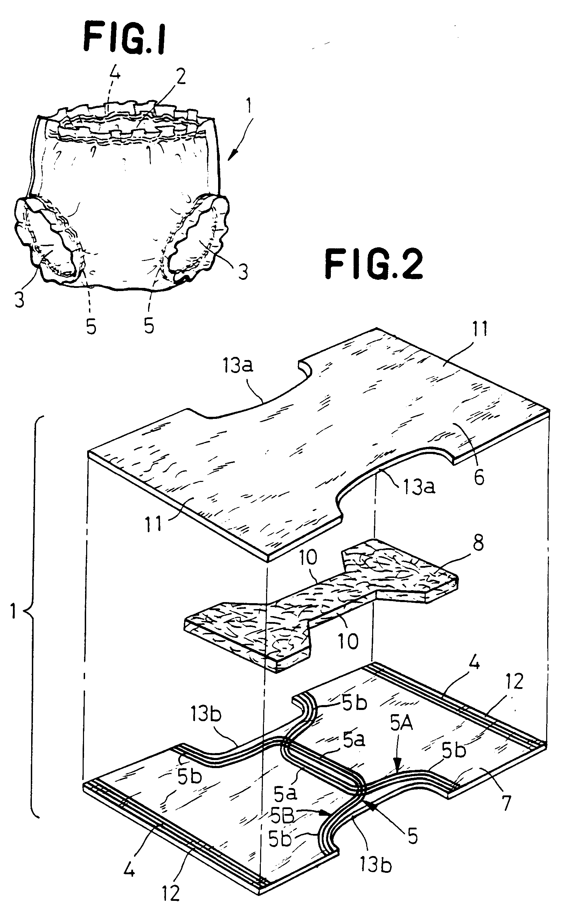

Fig. 1 ist eine perspektivische Darstellung einer Wegwerfunterhose, die entsprechend des erfindungsgemäßen Verfahrens hergestellt und vollständig zusammengesetzt ist. Die Unterhose 1 weist eine Taillenöffnung 2, ein Paar Beinöffnungen 3 und elastische Teile 4,5 auf, die um die Öffnungen herum an den Unterhosen 1 angeklebt sind, und um diese Öffnungen herum elastische Falten ausbilden.

Fig. 2 ist eine perspektivische Expolosionsdarstellung der bezeichneten Unterhosen. Wie aus dieser Figur zu ersehen ist, enthält diese Unterhose 1 eine obere Lage 6 und eine rückwärtige Lage 7, die jeweils aus einem Faservlies besteht, das sowohlin Längs- als auch in Querrichtung elastisch ist, mit einem mattenartigen Saugkern 8, der zwischen diesen beiden Lagen eingeschlossen ist, und den genannten elastischen Teilen 4,5. Die obere Lage 6 und die rückwärtige Lage 7 sind jeweils an einander gegenüberliegenden Seiten mit Ausschnitten 13a,13a und 13,13b versehen. Der Kern 8 ist ebenfalls an einander gegenüberliegenden Seiten mit Ausschnitten 10,10 ausgestattet, so daß er eine sogenannte Sanduhrform besitzt. Des weiteren besteht der Kern 8 hauptsächlich aus flockiger Pulpe. Das elastische Teil 4 ist seitlich elastisch zwischen der Oberlage 6 und der Rücklage 7 entlang der Taillenbereiche 11,12 mittels eines Klebers befestigt, was nachfolgend im Detail erläutert werden wird. Das elastische Teil 5 weist erste und zweite Teilelemente 5A,5B auf, die ihrerseits aus drei Gummifäden mit Zentralabschnitten 5a,5a, und gegenüberliegenden Seitenabschnitten 5b,5b bestehen. Diese Gummifäden sind zwischen der Oberlage 6 und der rückwärtigen Lage 7 lediglich an ihren einander gegenüberliegenden Seitenabschnitten 5a,5b befestigt.

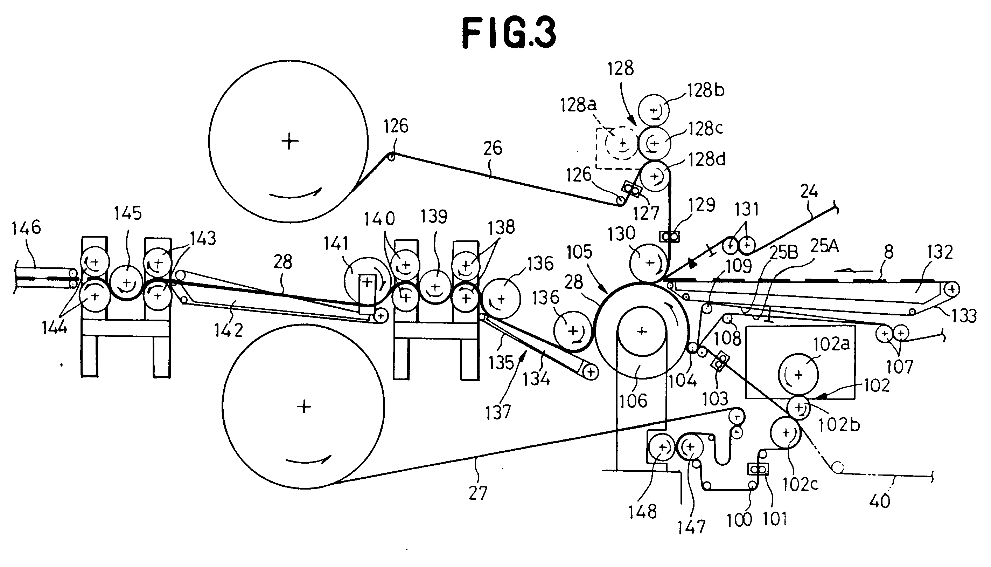

Fig.3 zeigt schematisch eine Vorrichtung zur Herstellung von Unterhosen. Eine kontinuierliche Bahn 27 als Ausgangsmaterial für die rückseitige Lage 7 wird von einer Gruppe Führungswalzen 100 und einem Zugwalzenpaar 101 zum Straffen der genannten Bahn in Querrichtung in eine Klebeauftragsstation 102 geführt, bestehend aus Walzen 102a,102b und 102c. An der Auftragsstation 102 wird die Bahn 27 mittig mit heißschmelzendem Klebstoff versehen, um ovale Klebmittelzonen 30a herzustellen, die geringfüg in Querrichtung der Bahn 27 gestreckt sind und voneinander in Längsrichtung der Bahn 27 einen regelmäßigen Abstand aufweisen. Die Bahn 27 wird dann von einem Zugrollenpaar 103, die ausgelegt sind, die Bahn 27 in Querrichtung zu spannen, zu einem Quetschwalzenpaar 104 und dann zu einer drehbaren Trommel 106 in einer Zusammenbaustation 105 geführt.

Drei kontinuierliche elastische Teile 25A,25B werden als die elastischen Teilelemente 5A,5B von einem Dehnwalzenpaar 107 gedehnt, wenn sie zu den in Querrichtung wirkenden Mitteln 108,109 geführt werden.

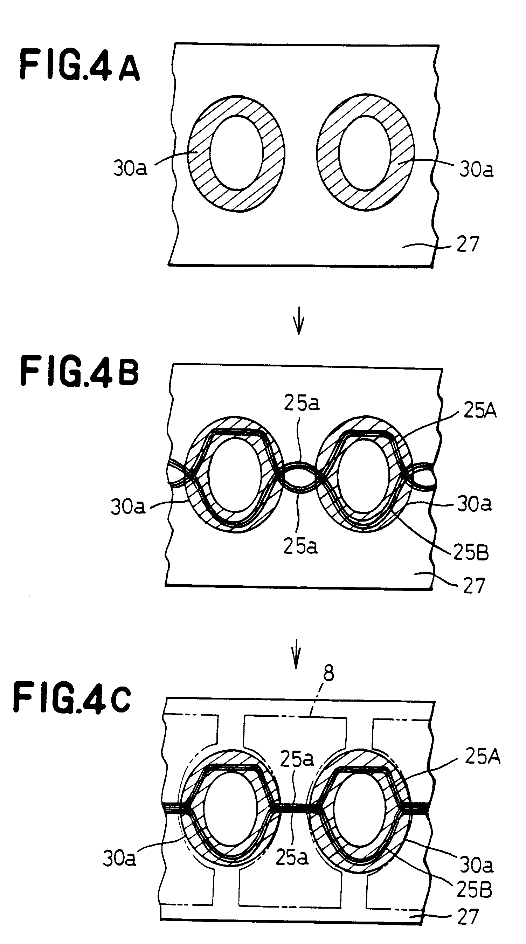

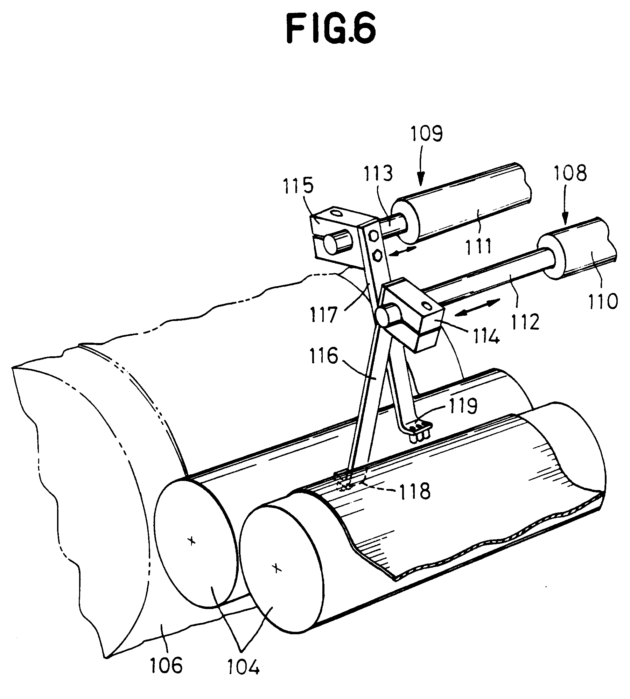

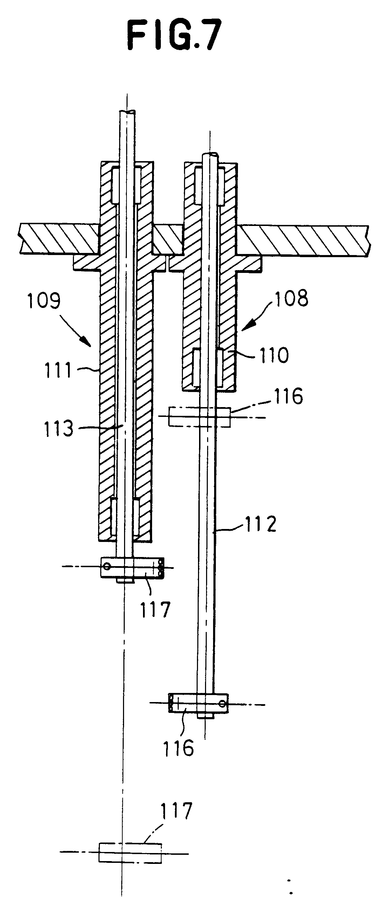

Die Figuren 6 und 7 zeigen jeweils in perspektivischer Ansicht und im Schnitt Anordnungungen der in Querrichtung wirkenden Mittel 108,109. Die in Querrichtung wirkenden Mittel 108,109 weisen jeweils Trägerzylinder 110,111 auf, die nebeneinander und parallel zu dem Quetschwalzenpaar 104 liegen, mit verschiebbaren Stangen 112,113, die in diesen Trägerzylindern eingeschoben sind und mit Führungsstangen 116,117 ausgestattet sind, die von Trägerblocken 114,115 herabhängen, welche ihrerseits jeweils an den vorderen Enden der verschiebbaren Stangen 112,113 befestigt sind. Die genannten verschiebbaren Stangen 112,113 werden von nicht dargestellten Quernocken gesteuert, die jeweils an den Basisenden dieser Stangen 112,113 vorgesehen sind. Die Führungsstangen 116,117 sind an ihren unteren Enden mit Führungsbohrungen 118,119 ausgestattet, durch welche hindurch die elastischen Teile 25A,25B jeweils hindurchgeführt werden. Die unteren Enden der Führungsstangen 116,117 sind der Umfangsoberfläche des Quetschwalzenpaars 104 eng benachbart. Die verschiebbaren Stangen 112,113 sind über Distanzen hinweg zwischen Positionen der jeweiligen Führungsstangen 116,117, angezeigt mittels durchgezogener Linien und Stellungen der genannten Stangen 116,117, angezeigt durch unterbrochene Linien in Fig. 7 und gesteuert von den Quernocken bewegbar. Wenn die verschiebbaren Stangen 112,113 und demzufolge die Führungsstangen 116,117 bei einer derartigen Steuerung bewegt werden, werden die elastischen Teile 25A,25B, die feiner sind als die Klebmittelstreifen, welche die genannten Klebmittelzonen 30A auf der Bahn 27 bilden, aufgelegt um sich auf der wandernden Bahn mit den genannten Klebmittel-Zonen 30 zu verbinden, so daß die genannten elastischen Teile 25A,25B im wesentlichen der Hälfte eines jeden Paares der benachbarten Klebzonen 30a folgen, sich dann gegenseitig in dem Bereich schneiden , der zwischen diesen nebeneinanderliegenden Klebzonen 30a definiert sind und schließlich den verbleibenden Hälften folgen, wobei sie ein Paar Sinuskurven beschreiben , wie in Fig. 4B dargestellt ist.

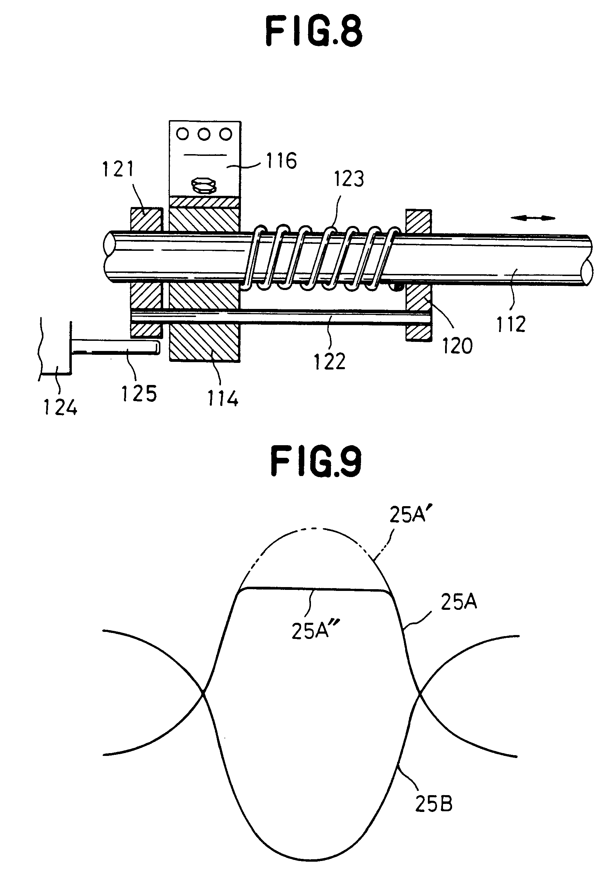

Fig. 8 ist eine teilweise Schnittansicht, in der die Mechanik dargestellt ist, die die Bewegung der verschiebbaren Stange 112 und der Führungsstange 116 steuert und dabei teilweise die von dem genannten elastischen Teil 25A beschriebene Sinuskurve deformiert. In Fig. 9 ist eine Draufsicht auf die Sinuskurve dargestellt, die in dieser Ansicht teilweise deformiert ist. Es sei erneut Bezug auf Fig. 8 genommen, worin neben dem vorderen Ende des Trägerzylinders 110 aus Fig. 6 auf der verschiebbaren Stange 112 ortsfest ein Paar Regulierblöcke 120,121 im Abstand voneinander entlang der Längserstreckung der bewegbaren Stange 112 angeordnet sind. Auf dem Abschnitt der verschiebbaren Stange zwischen den Regulierblöcken 120,121 ist ein Trägerblock 114 verschiebbar angeordnet. Zwischen den beiden Regulierblöcken und durch den Trägerblock 114 hindurchführend, ist noch eine Führungsstange 122 vorgesehen. Zwischen dem Trägerblock 114 und dem Regulierblock 120 ist eine Schraubenfeder 123 unter Spannung eingesetzt. Ein Stopper 125 erstreckt sich von einem stationären Element 124 derart, daß er an der gegenüberliegenden Oberfläche des Trägerblocks 114 zur Anlage kommt und dabei dessen Bewegung begrenzt. Wenn die verschiebbare Stange 112 nicht mit einem solchen Mechanismus ausgestattet wäre, würde das elastische Teil 125 eine Sinuskurve mit einem kreisförmigen Abschnitt 25A'beschreiben, wenn die bewegbare Stange 112 und die Führungsstange 116 bewegt werden, wohingegen im Falle, wie in Fig. 8 dargestellt, in dem die verschiebbare Stange 112 mit einer solchen Mechanik ausgestattet ist, bei Kontakt des Trägerblocks 114, der die Führungsstange 116 trägt, mit dem Stopper 125 die genannte Sinuskurve des elastischen Teils 25 deformiert ist, so daß diese einen geradlinigen Abschnitt 25A'' beschreibt, entsprechend der Zeitdauer, während der die Führungsstange 116 zeitweilig durch den Stopper 125 angehalten wird, der gegen den Trägerblock 114 stößt. Eine derart deformierte Sinuskurve des elastischen Teils 25A, ist in Übereinstimmung mit den Konfigurationen der Beinöffnungen 3, dargestellt in Fig. 1 und dem Schrittbereich besonders ausgelegt, um die Paßform zu verbessern.

Die Lage 27 einschl. der elastischen Teile 25A, 25B die in der oben beschriebenen Weise auf die Bahn gelegt wurden, wird zwischen dem Quetschwalzenpaar 104 zusammengedrückt, wobei die elastischen Teile sich klebend mit der Bahn verbinden. An diesem Punkt ziehen sich die Abschnitte 25a,25a, der jeweiligen elastischen Teile 25A,25B, die nicht in den Klebzonen 30a liegen zusammnen, um eine im wesentlichen geradlinige Gestalt anzunehmen, wie in Fig. 5C dargestellt.

Es wird noch einmal Bezug auf Fig. 3 genommen, worin eine kontinuierliche Bahn 26 als Material für die genannte obere Lage 6 von einer Führungswalze 126 geführt und ein Zugwalzenpaar 127 vorgesehen ist, das diese Bahn 26 in Querrichtung strafft, wobei die Bahn zu einer Klebemittelauftragsstation 128 geführt wird, die Walzen 128a,128b und 128d aufweist. In dieser Auftragsstation 128 wird die Bahn im Zentralbereich mit einem heißschmelzendem Klebmittel versehen, um ovale Klebmittelzonen 30b zu schaffen, welche in Querrichtung der Bahn 26 geringfügig gestreckt sind, wie in Fig. 4D dargestellt. Die Gestalt, die Größe und der Abstand dieser Klebmittelzone 30b sind im wesentlichen identisch mit der vorausgehend erwähnten Klebzone 30a. Gleichzeitig wird die Bahn 26 mit geradlinigen Klebzonen 31 versehen, die sich von einander gegenüberliegenden Seiten zu den jeweils danebenliegenden Seitenrändern der Bahn 26 erstrecken, und mit kontinuierlichen geradlinigen Klebmittelzonen 32, die sich entlang der Seitenränder der Bahn 26 in Längsrichtung erstrecken. Dieses Auftragen des heißschmelzenden Klebmittels geschieht in der Auftragsstation 128. Anschließend wird die Bahn 26 mittels eines Zugwalzenpaars 129, das ebenfalls ausgelegt ist, die Bahn 26 in Querrichtung zu straffen, zwischen die rotierende Trommel 106 und einer Druckwalze 130 gefördert, welche mit der genannten Trommel zusammenwirkt.

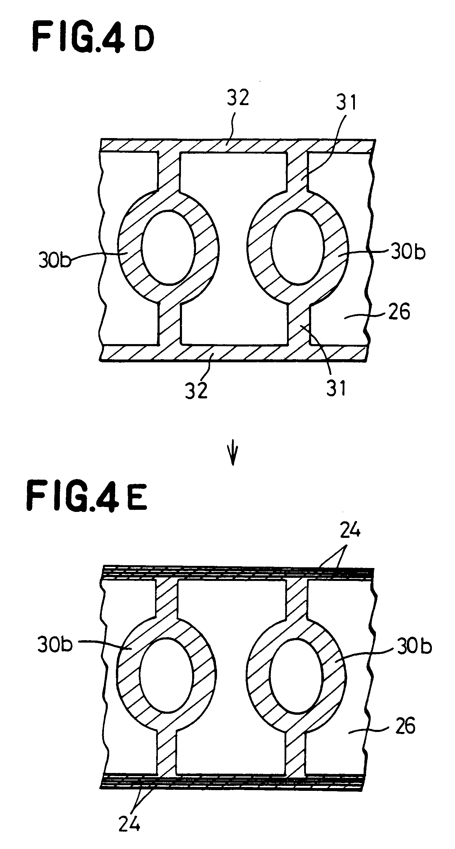

Die kontinuierlichen elastischen Teile 24 als Material für die elastischen Teile 4 werden bis zu einem gewünschten Verhältnis gedehnt und gleichzeitig durch ein Dehnwalzenpaar 131 geführt, um sie entlang der Klebmittelzonen 32, die entlang der Seitenränder der Lage 26 ausgeformt sind, aufzulegen und anzukleben, vergl. Fig. 4E.

In Fig. 3 ist zu erkennen, daß die individuellen Saugkerne 8 die vorgeformt sind, in regelmäßigen Abständen auf einem perforierten Bandförderer 133 aufgelegt werden und über einer Vorrichtung 132 bewegt werden, welche eine relativ schwache Saugwirkung ausübt; mittels des Bandförderers 133 werden diese Kerne zur Zusammenbaustation 105 gefördert. Wie aus den Figuren 2 und 4C (angezeigt durch unterbrochene Linien) hervorgeht, weist jeder Kern 8 eine sogen. Sanduhrgestalt auf und ist jeweils zwischen jedem Paar einander gegenüberliegender Klebmittelzonen 30a oder jedem Paar benachbarter-Schleifenabschnitte angeordnet, die jeweils von den elastischen Teilen 25A,25B auf der Bahn 27 ausgeformt sind. Auf die Bahn 27, die den darauf abgelegten Saugkern 8 trägt, wird die Bahn 26 so aufgelegt, daß die Klebmittelzonen 30a,30b auf den entsprechenden Klebmittelzonen 30b,30b zu liegen kommen. Auf diese Weise werden die jeweiligen Schleifenabschnitte der genannten elastischen Teile 25A,25B genau sandwichartig zwischen die Bahnen 26 und 27 an den Klebmittelzonen 30a,30b eingeschlossen und die elastischen Teile 24 werden ebenfalls genau sandwichartig zwischen diesen Bahnen 26,27 entlang ihrer seitlichen Ränder eingeschlossen. Daraufhin werden die Bahnen 26,27, die die Saugkerne 8 und die elastischen Teile 24,25A,25B zwischen sich einschließen zwischen der drehbaren Trommel 106 und der Druckwalze 130 zusammengedrückt, um die kontinuierliche kombinierte Bahn 28 zu bilden und gleichzeitig die Kerne und die elastischen Teile 24,25A,25B in dieser kombinierten Lage 28 zu fixieren.

Die kombinierte Lagenbahn 28, die die Kerne 8 einschließt, wird dann einer Preßstation, die einen Bandförderer 35 aufweist, der auf dem oberen Abschnitt eines Laufgerüsts 134 läuft und Druckwalzenpaare 136 und anschließend einem Heißschweißwalzenpaar 138 zugeführt. Bei diesem Walzenpaar kann es sich auch um eine Apparatur handeln, die mit Ultraschall arbeitet. Die kombinierte Lagenbahn 28 wird an ihren ovalen Zonen 21, definiert durch jedes Paar nebeneinanderliegender Saugkerne 8 und geradliniger Abschnitte 22, die sich voneinander gegenüberliegenden Seiten der jeweiligen ovalen Zonen 21 zu nebenliegenden seitlichen Rändern der kombinierten Lage 28 erstrecken, mittels des Heißschweißwalzenpaares 138 heiß verschweißt, vergl. Fig. 5A. In diesem Fall ist eine besonders harte Heißverschweißung nicht wünschenswert. Es versteht sich, daß dieser Vorgang nicht notwendig ist für die Erfindung.

Die kombinierte Lagenbahn 28, die auf diese Weise hergestellt wurde, wird nun mittels Führungswalzen 139 einem Walzenschneider 140 zugeführt, mittels welchem ein schraffierter Zentralbereich 21' der ovalen Zone 21 ausgeschnitten wird, um die Ausschnitte 23 für die Beinöffnungen zu bilden. Der Zentralbereich 21' umfaßt Abschnitte der Lagen 26,27, welche innerhalb der durch die Klebmittelzonen 30a,30b und der elastischen Teile 25A, 25A, die an diesen Zonen entlang angeklebt sind, liegen.

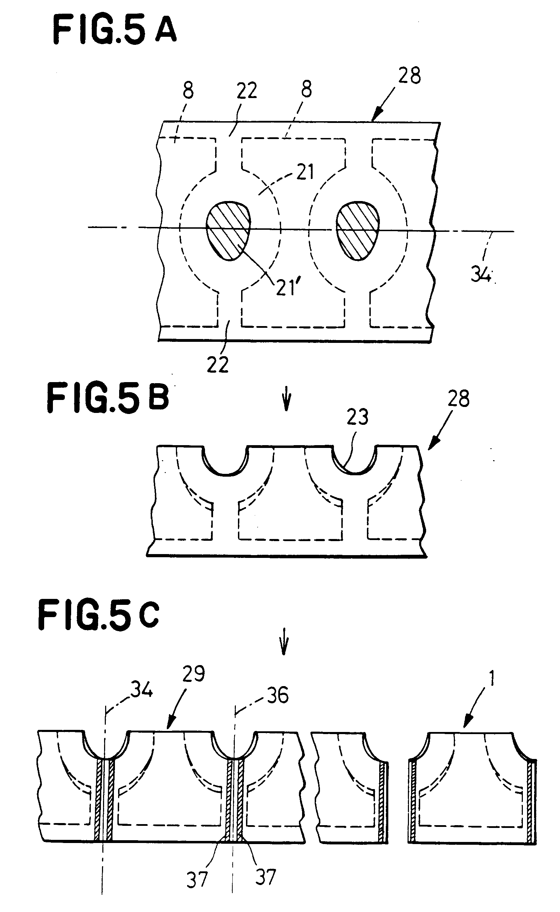

Die kombinierte Lagenbahn 28, die auf diese Weise mit den Ausschnitten 23 versehen ist, wird mittels einer Führungswalze 141 zu Faltmitteln 142 geleitet, deren Details nicht dargestellt sind. Die Faltmittel 142 können eine bekannte Anordnung sein, wie sie üblicherweise bei Anlagen zur Herstellung von Wegwerfwindeln, Binden oder ähnlichem verwendet wird. Die kombinierte Lagenbahn 28 wird mittels der Faltmittel 142 entlang einer längsverlaufenden Mittellinie 34, wie in Fig. 5A dargestellt, in zwei Hälften gefaltet, vergl. Fig. 5B.

Die gefaltete kombinierte Lagenbahn 28 wird dann zu einer Heiß- oder (Ultraschall) Schweißwalzenpaar 143 geführt, wo sie mit geraden oder bandförmigen Heißschweißzonen 37 versehen wird, die sich neben imaginärer Schnittlinien 36 auf gegenüberliegenden Seiten der individuellen Unterhosen 1 erstrecken, wodurch eine kontinuierliche Serie von Unterhosen 29 ausgebildet wird.

Die kontinuierliche Serie von Unterhosen 29 wird mittels einer Führungswalze 145, einer Schnittwalze 144 zugeführt, welche die genannte kontinuierliche Serie von Unterhosen schrittweise entlang der imaginären Schnittlinien 36 oder an den Heißschweißzonen 37 in individuelle Unterhosen 1 schneidet, welche dann mittels eines Bandförderers 146 zu einem Verpackungsprozeß (nicht dargestellt) transportiert werden.

Wenn eine flüssigkeitsundurchlässige Lage als kontinuierliche Bahn 27 verwendet wird, welche das Material für die rückseitige Lage 7 bildet und es gewünscht ist, zumindest die Zentralzone der rückwärtigen Lage, bei der der Kern 8 auf der rückwärtigen Lage angeordnet ist, undurchlässig zu machen, um jede Möglichkeit eines Ausfließens von Körperflüssigkeit durch die zentrale Zone zu verhindern, wird es bevorzugt, die flüssigkeitsundurchlässige Bahn mit der Bahn 27 vor dem Aufbringen der elastischen Teile 25A, 25B zu verkleben. Die flüssigkeitsundurchlässige Lage besitzt oder ist aus vorzugsweise einen elastisch gemachten Kunststoffilm oder etwas ähnliches. In einem solchen Fall, es sei hier wieder Bezug auf Fig. 3 genommen, wird die Lage 27 zentral mit einem heißschmelzbaren Klebmittel mit punktverteiltem Muster versehen, wenn sie zwischen einer Druckwalze 127 und einer Auftragwalze 148 läuft, so daß eine flüssigkeitsundurchlässige, kontinuierliche Bahn 40 mit diesem Zentral bereich verklebt werden kann. Dementsprechend wird das Auftragen von Klebmitteln in der Auftragsstation 102 wie auch das Aufbringen von den elastischen Teilen 25A,25B in diesem Fall teilsweise sowohl auf der Bahn 40 als auch auf der Bahn 27 durchgeführt.

Vorzugsweise bestehen die Bahnen 26,27 aus Faservlies, das sowohl in der Breite, als auch in der Länge elastisch ist oder zumindest in der Breite; der Saugkern 8 besteht aus flockiger Pulpe, die mit supersaugfähigem Polymerpuder vermischt und miteinander verschmolzen ist, und die elastischen Teile 24,25A,25B bestehen aus natürlichem oder synthetischen Gummifäden oder Bändern oder Kunststoffilmen, die nach Heißbehandlung elastisch werden. Der Kern 8 kann abhängig von der besonderen Verwendung der Unterhosen auch weggelassen werden.

Es versteht sich, daß der Schritt der Fig. 5A durch den Schritt der Fig. 5B ersetzt werden kann, um den Zentralbereich 21' der kombinierten Lage 28 auszuschneiden, um den Ausschnitt 23 für die Beinöffnung auszuformen. Des weiteren können die Klebzonen 30A,30B,31 und 32 mit Klebmitteln kontinuierlich sich über die gesamten Zonen erstreckend beaufschlagt werden, oder mit einer Vielzahl von punktförmigen Aufträgen intermittierenden Linien oder Schraubenlinien.

## Patentansprüche

1. Verfahren zum Herstellen von Wegwerfunterhosen mit folgenden Verfahrensschritte :
a) Intermittierendes Auftragen (102) von ringförmigen Klebzonen (30a) zentral auf einer Seite einer ersten kontinuierlichen Bahn (27) entlang ihrer Länge;
b) Zuführen von ersten (25A) und zweiten (25B) kontinuierlichen elastischen Teilen, die eine Breite kleiner als diejenige eines Klebstreifens aufweisen (30A), welcher die genannten ringförmigen Klebezonen in Richtung auf den Zentralbereich auf der genannten einen Seite der genannten ersten kontinuierlichen Bahn (27) definiert, so daß das genannte erste kontinuierliche elastische Teil (25A) so aufgelegt ist, daß es entlang im wesentlichen einer Hälfte der genannten ringförmigen Klebzone (30a) anklebt, während das genannte zweite kontinuierliche elastische Teil (25B) so aufgelegt wird, daß es entlang im wesentlichen der verbleibenden Hälfte der genannten ringförmigen Klebzone (30a) anhaftet;
c) Vorsehen von bandartigen Klebzonen (32), die sich entlang seitlicher Ränder auf einer Seite zumindest einer der genannten ersten (27) und einer zweiten (26) kontinuierlichen Bahn befinden;
d) Einführen einer dritten Gruppe kontinuierlicher elastischer Teile (24) zwischen den einander gegenüberliegenden Seiten der genannten ersten kontinuierlichen Bahn (27) und der zweiten kontinuierlichen Bahn (26) entlang deren jeweiligen Seitenränder, so daß sie zwischen diesen Bahn (26,27) entlang der genannten bandartigen Klebezonen (32) ankleben;
e) Verkleben der genannten ersten (27) und zweiten (26) kontinuierlichen Bahn zusammen entlang der bezeichneten ein ander gegenüberligenden Seiten, um eine kontinuierliche kombinierte Bahn (28) zu bilden;
f) Wegschneiden von Abschnitten (21') der genannten kontinuierlichen kombinierten Bahn (28), die in den jeweiligen ringförmigen Zonen (21), die diese ringförmigen Klebzonen (30a,30b) aufweisen, eingeschlossen sind, wobei die ersten (25A) und zweiten (25B) elastischen Teile, die entlang der genannten ringförmigen Klebzone (30a,30b) auf die Bahn geklebt sind nach dem Formen oder Falten die genannte kombinierte Bahn (28) doppeln, um Ausschnitte (23) für die Beinöffnungen zu bilden;
g) Falten der kontinuierlichen kombinierten Bahn (28) zu zwei Hälften entlang einer in Längsrichtung verlaufenden Zentrallinie;
h) Anlegen von geradlinigen Abdichtzonen (37), die sich in Querrichtung der doppelt gefalteten kontinuierlichen kombinierten Bahn (28) zentral durch die jeweiligen Ausschnitte (23) für die Beinöffnungen zu einer Seitenkante der kombinierten Bahn erstrecken, entsprechend der Taillen-Linie, um eine kontinuierliche Serie von Unterhosen (29) zu bilden; und
i) Schneiden der kontinuierlichen Serie von Unterhosen (29) entlang jeder dieser geraden Dichtzonen (37) in Querrichtung zur kontinuierlichen Serie der Unterhosen, so daß jede der linearen Dichtzonen (37) in Längsrichtung der genannten kontinuierlichen kombinierten Bahn zweigeteilt ist und auf diese Weise individuelle Unterhosen erhalten werden.

2. Verfahren zum Herstellen von Wegwerfunterhosen nach Anspruch 1, wobei während oder nach dem Schritt "c" die zweite kontinuierliche Bahn auf einer Seite mit zweiten ringförmigen Klebzonen versehen wird, damit sie jeweils mit den zuerst erwähnten ringförmigen Klebzonen übereinstimmt und wobei die zweiten ringförmigen Klebzonen mit den ersten und zweiten kontinuierlichen elastischen Teilen verklebt werden, die an den ersten ringförmigen Klebzonen befestigt sind.

3. Verfahren zum Herstellen von Wegwerfunterhosen nach Anspruch 1 oder 2, wobei die ersten und zweiten kontinuierlichen elastischen Teile sich gegenseitig an gegenüberliegenden Enden der jeweiligen ringförmigen Klebzonen überschneiden, betrachtet in Längsrichtung der Bahn.

4. Verfahren zum Herstellen von Wegwerfunterhosen nach Anspruch 1 oder 2, wobei ein Saugkern (8) zwischen jedem Paar nebeneinanderliegender ringförmiger Klebzonen eingesetzt wird, bevor die genannten ersten und zweiten Bahnen aufeinander gelegt werden.

5. Verfahren zum Herstellen von Wegwerfunterhosen nach Anspruch 1 oder 2, wobei elastisches Faservlies als erste und zweite kontinuierliche Bahn verwendet wird.

## Claims

1. Process for the manufacture of disposable underpants with the following process steps:
a) intermittent application (102) of annular adhesive zones (30a) centrally on one side of a first continuous line (27) along the length thereof;
b) leading first (25A) and second (25B) continuous elastic parts with a width smaller than that of an adhesive stripe (30A) which defines said annular adhesive zones in the direction of the central part of said one side of said first continuous line (27), so that said first continuous elastic part (25A) is so laid that it sticks along essentially one half of said annular adhesive zone (30a), while said second continuous elastic part (25B) is so laid that it is secured to essentially the remaining half of said annular adhesive zone (30a);
c) provision of band-like adhesive zones (32) along lateral edges of one side of at least one of said first (27) and a second (26) continuous line;
d) introduction of a third group of continuous elastic parts (24) between the opposite sides of said first continuous line (27) and the second continuous line (26) along their respective side edges, so that they stick between these lines (26,27) along said band like adhesive zones (32);
e) sticking said first (27) and second (26) continuous lines together along the designated opposing sides in order to form a continuous combined line (28);
f) cutting off portions (21') of said continuous combined line (28) which are included in the respective annular zones (21) with the annular adhesive zones (30a,30b), whereby the first (25A) and second (25B) elastic parts, which are stuck to the line along said annular adhesive zones (30a,30b), are doubled after forming or folding of said combined line (28) to form cut outs (23) for the leg openings;
g) folding the continuous combined line (28) into two halves along a longitudinally extending central line;
h) application of linear sealing zones (37), which extend perpendicularly to the double folded continuous combined line (28) centrally through the respective cut outs (23) for the leg openings to a side edge of the combined line, corresponding to the waistline, to form a continuous series of underpants (29); and
i) cutting the continuous series of underpants (29) along each of these linear sealing zones (37) in a direction perpendicular to the continuous series of underpants, so that each of the linear ceiling zones (37) is cut in two in the longitudinal direction of said continuous combined line and in this way individual underpants are obtained.

2. Process for the manufacture of disposable underpants according to Claim 1, whereby during or after step "c" the second continuous line is provided on one side with second annular adhesive zones such that they correspond to the first mentioned annular adhesive zones and whereby the second annular adhesive zones are stuck to the first and second continuous elastic parts which are secured to the first annular adhesive zones.

3. Process for the manufacture of disposable underpants according to Claim 1 or 2, whereby the first and second continuous elastic parts overlap at opposite ends of the respective annular adhesive zones, when viewed in the longitudinal direction of the line.

4. Process for the manufacture of disposable underpants according to Claim 1 or 2, whereby an absorbent body (8) is interposed between each pair of adjacent annular adhesive zones before said first and second lines are laid on each other.

5. Process for the manufacture of disposable underpants according to Claim 1 or 2, whereby elastic fibre fleece is used as first and second continuous line.

## Revendications

1. Procédé pour la fabrication de couches-culottes jetables, dont les étapes de fabrication sont les suivantes :
a) Etalement par intermittence (102) de zones de colle (30a) circulaires centrées sur la longueur d'une face d'une première bande continue (27) ;
b) Mise en place d'un premier (25A) et d'un deuxième (25B) éléments élastiques continus, qui présentent une largeur inférieure à celle d'une traînée de colle (30a) et épousent les zones de colles circulaires indiquées en direction du centre sur la face indiquée de la première bande continue désignée par (27), et ce de manière que le premier élément élastique continu désigné par (25A) soit posé et se colle pour l'essentiel le long d'une moitié de la zone de colle circulaire désignée par (30a), tandis que le deuxième élément élastique continu mentionné (25B) est posé de manière à adhérer pour l'essentiel sur l'autre moitié de la zone de colle circulaire désignée par (30a) ;
c) Apposition de bandes de colle (32) le long des bords latéraux sur une face au moins de la première bande continue désignée par (27) et d'une deuxième bande continue (26) ;
d) Placement d'un troisième groupe d'éléments élastiques continus (24) entre les faces opposées de la première bande continue mentionnée (27) et de la deuxième bande continue (26) le long des bords de chaque face, de manière que ces éléments puissent se coller entre ces bandes (26,27) le long des bandes de colles désignées par (32) ;
e) Collage réciproque de la première bande continue mentionnée (27) contre la deuxième (26) le long des côtés opposés indiqués, en vue de constituer une bande combinée continue (28) ;
f) Découpage dans la bande combinée continue désignée par (28), des segments (21') inclus dans chaque zone circulaire (21), qui comportent ces zones de colle circulaires (30a,30b), tandis qu'après le pliage ou la mise en forme, le premier (25A) et le deuxième (25B) éléments élastiques, qui sont collés le long des zones de colle circulaires désignées par (30a,30b), doublent la bande combinée mentionnée (28), pour former les passages (23) pour les jambes ;
g) Pliage en deux de la bande combinée continue (28) le long de la ligne longitudinale médiane ;
h) Réalisation, en fonction de la taille, de zones rectilignes étanches (37), qui, transversalement à la bande combinée continue (28) repliée en double, passent par le milieu des passages respectifs (23) pour les jambes et constituent une arête latérale pour la bande continue, et ceci en vue de former une série de couches-culottes (29) ; et
i) Découpage de la série continue de couches-culottes (29) le long de chacune de ces zones rectilignes étanches (37) en direction transversale de cette série, si bien que chacune des zones rectilignes étanches (37) est, sur la direction longitudinale de la bande combinée mentionnée, coupée en deux, ce qui fait que l'on obtient ainsi des couches-culottes individuelles.

2. Procédé pour la fabrication de couches-culottes jetables selon la revendication 1, tandis que, pendant ou après l'étape "c", la deuxième bande continue est pourvue sur une face de deuxièmes zones circulaires de colle, qui concordent respectivement avec les premières zones circulaires de colle, les deuxièmes zones circulaires de colle étant collées avec les premier et deuxième éléments élastiques continus fixés sur les premières zones circulaires de colle.

3. Procédé pour la fabrication de couches-culottes jetables, selon la revendication 1 ou 2, les premier et deuxième éléments élastiques continus, vus en direction longitudinale de la bande, se chevauchant mutuellement aux extrémités opposées de chaque zone circulaire de colle.

4. Procédé pour la fabrication de couches-culottes jetables selon la revendication 1 ou 2, un noyau absorbant (8) étant introduit entre chaque paire de zones circulaires de colle contiguës, avant que les première et deuxième bandes mentionnées ne soient appliquées l'une sur l'autre.

5. Procédé pour la fabrication de couches-culottes jetables selon la revendication 1 ou 2, du tissu élastique étant utilisé pour la première et la deuxième bandes continues.
